(19) Europäisches Patentamt
European Patent Office
Office européen des brevets

(11) Veröffentlichungsnummer: **0 065 733**
**B1**

# (12) EUROPÄISCHE PATENTSCHRIFT

(45) Veröffentlichungstag der Patentschrift: **11.07.84**

(51) Int. Cl.³: **C 07 C 91/44,** C 07 C 89/00

(21) Anmeldenummer: **82104302.3**

(22) Anmeldetag: **17.05.82**

(54) **Verfahren zur Herstellung von 3-Alkylaminophenolen.**

(30) Priorität: **21.05.81 DE 3120245**

(43) Veröffentlichungstag der Anmeldung:
**01.12.82 Patentblatt 82/48**

(45) Bekanntmachung des Hinweises auf die Patenterteilung:
**11.07.84 Patentblatt 84/28**

(84) Benannte Vertragsstaaten:
**BE CH DE FR GB IT LI NL**

(56) Entgegenhaltungen:
**DE - A - 2 402 695**

**Chemical Abstracts Band 88, Nr. 7 13. Februar 1978 Columbus, Ohio, USA I.G. TISHCHENKO et al. "o-Aminophenols" Seite 511, Spalte 2, Abstract Nr. 50473u**

(73) Patentinhaber: **HOECHST AKTIENGESELLSCHAFT, Postfach 80 03 20, D-6230 Frankfurt am Main 80 (DE)**

(72) Erfinder: **Reimann, Walter, Dr., Loreleistrasse 46, D-6230 Frankfurt am Main 80 (DE)**
Erfinder: **Müller, Werner Heinrich, Dr., Taunusblick 5, D-6239 Eppstein/taunus (DE)**

**EP 0 065 733 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft ein Verfahren zur Herstellung von 3-Alkylaminophenolen durch katalytische Dehydrierung von 3-Alkylamino-2-cyclohexenonen in der Gasphase.

3-Alkylaminophenole sind wichtige Zwischenprodukte für Arzneimittel, Farbstoffe (DE-OS 24 58 347), Herbizide, optische Aufheller und Antioxidantien.

Die US-PS 4 212 833 beschreibt ein Verfahren zur Herstellung von 3-Aminophenolen, das dadurch gekennzeichnet ist, dass 3-Amino-2-cyclohexenone in Gegenwart eines Dehydrierungskatalysators in der Flüssigphase bei 150–300°C dehydriert werden. Dabei ist im allgemeinen ein inertes Lösungsmittel erforderlich, das zwischen 150 und 300°C siedet. Insbesondere werden Kohlenwasserstoffe und Polyglykolether verwendet. Zur Erzielung hoher Ausbeuten benötigt man eine Menge an Lösungsmittel, die 5 bis 10 mal so hoch ist wie die Menge an 3-Amino-2-cyclohexenon, und die entstehenden 3-Aminophenole müssen durch Destillation vom Lösungsmittel getrennt werden.

Nachteilig bei diesem Verfahren ist die Belastung durch grosse Lösungsmittelmengen, die zum Teil bei den Reaktionstemperaturen leicht entzündlich sind.

Es wurde nun gefunden, dass 3-Alkylamino-2-cyclohexenone, insbesondere das 3-Dimethylamino-2-cyclohexenon, entweder allein oder in Verbindung mit einem Schleppmittel verdampft werden können und sich in der Gasphase an Dehydrierungskatalysatoren zu 3-Alkylaminophenolen dehydrieren lassen.

Die Erfindung betrifft somit ein Verfahren zur Herstellung von 3-Alkylaminophenolen der allgemeinen Formel I

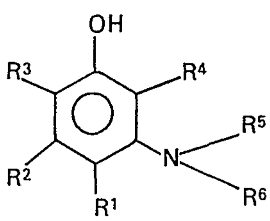

(I)

wobei die Reste $R^1$ bis $R^5$ unabhängig voneinander Wasserstoff, Alkyl- oder Cycloalkylgruppen mit bis zu 12 C-Atomen und gegebenenfalls halogensubstituiert sein können und $R^6$ eine Alkyl- oder Cycloalkylgruppe mit bis zu 12 C-Atomen oder zusammen mit $R^5$ einen Alkylenring mit 4 bis 6 C-Atomen, welcher noch ein Sauerstoff- oder ein substituiertes Stickstoff-Atom enthalten kann, bedeutet, durch katalytische Dehydrierung von 3-Amino-2-cyclohexenonen der allgemeinen Formel II:

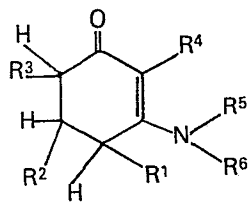

(II)

wobei die Reste $R^1$ bis $R^6$ die oben angegebene Bedeutung haben, dadurch gekennzeichnet, dass man die Dehydrierung in der Gasphase bei 200 bis 500°C und einem Druck von 0.1 bis 10 bar durchführt.

Die für die Reste $R^1$ bis $R^6$ geeigneten Alkylgruppen können dabei geradkettig, verzweigt oder cyclisch sein und im Falle von $R^1$ bis $R^5$ noch durch Halogenatome substituiert sein.

Die als Ausgangsverbindung für das erfindungsgemässe Verfahren benötigten 3-Alkylamino-2-cyclohexenone sind leicht zugänglich durch Umsetzung der entsprechenden 1,3-Cyclohexandione mit primären oder sekundären Aminen (US-PS 4 212 823).

Vorzugsweise sind die Reste $R^1$ bis $R^4$ Wasserstoff, Methyl, Ethyl, Propyl oder Butyl, insbesondere Wasserstoff oder Methyl. $R^5$ und $R^6$ sind vorzugsweise Methyl oder Ethyl.

Als Dehydrierungskatalysatoren werden Ruthenium, Rhodium, Palladium, Osmium, Iridium, Platin, Kupfer, Silber, Gold, Eisen, Kobalt oder Nickel verwendet, vorzugsweise Ruthenium, Rhodium, Palladium, Osmium, Iridium oder Platin. Als besonders günstig hat sich die Verwendung von Palladium erwiesen.

Diese Elemente werden im allgemeinen als Salze auf Trägermaterialien wie Kohle, Aluminiumoxid, Siliciumoxid, Chromoxid, Alumosilikate, Zeolite, Magnesiumoxid, Calciumoxid oder Titanoxid aufgebracht. Besonders bewährt haben sich Kohle und Siliciumoxid. Die Menge des aufgetragenen Metalls beträgt im allgemeinen zwischen 0,1 und 20 Gew.-%, vorzugsweise zwischen 0,1 und 5 Gew.-%. Die Trägerteilchen können eine beliebige äussere Form besitzen, vorzugsweise werden Kugeln oder Strangpresslinge verwendet.

Vor Beginn der Umsetzung ist es im allgemeinen zweckmässig, die auf den Träger aufgebrachten Salze durch Überleiten von Wasserstoff bei 250–500°C oder mittels anderer Reduktionsmittel zu reduzieren.

Die 3-Alkylamino-2-cyclohexenone können direkt verdampft werden. Zur sicheren Vermeidung teeriger Nebenprodukte während des Verdampfungsvorgangs ist es aber vorteilhaft, ein inertes Lösungsmittel, das zwischen 50 und 300°C siedet, als Schleppmittel für das Einsatzmaterial zu verwenden. Die Menge an Lösungsmittel beträgt im allgemeinen zwischen 10 und 200 Gew.-%, bezogen auf 3-Aminocyclohexenon, vorzugsweise zwischen 10 und 90 Gew.-%, insbesondere 20 bis 50 Gew.-%. Als Lösungsmittel sind z.B. geeignet: Wasser, aliphatische oder aromatische Kohlenwasserstoffe, wie Toluol und Dekalin, aliphatische und aromatische Ether, wie Diphenylether oder Diethylenglykoldimethylether.

Besonders geeignet ist Wasser: während die als Ausgangsmaterial verwendeten 3-Alkylamino-2-cyclohexenone in Wasser gut löslich sind, sind die bei der Dehydrierung entstehenden 3-Alkylaminophenole in Wasser weitgehend unlöslich. Dies bietet den Vorteil, dass die letzteren durch Abfiltrieren isoliert werden können und nach Trocknung sofort für eine weitere Verwendung zur Verfügung stehen. Das unumgesetzte 3-Alkylamino-2-cyclohexenon bleibt im Wasser gelöst und kann ohne weitere Reinigung wieder als Einsatzmaterial verwendet werden.

Bei Verwendung von Wasser als Schleppmittel läuft die Dehydrierung mit einer höheren Selektivität in bezug auf die gewünschten 3-Alkylaminophenole ab als bei Verwendung von organischen Lösemitteln. Es wird eine hohe Raum-Zeit-Ausbeute von bis zu 300 g/l·h erreicht. Überraschend ist, dass dabei das Wasser keine Hydrolyse der 3-Alkylamino-2-cyclohexenone bewirkt; insbesondere treten die denkbaren Nebenprodukte 1,3-Cyclohexandion und Resorcin nicht auf. Die Verwendung von Wasser statt der vorgenannten organischen Lösungsmittel ist auch vom Standpunkt der Betriebssicherheit vorzuziehen. Wasser kann auch zusammen mit organischen Lösemitteln, sofern sie mit Wasser mischbar sind, als Schleppmittel eingesetzt werden, z.B. Glykolethern.

Die Verdampfung der Ausgangsverbindung erfolgt vorzugsweise durch Auftropfen seiner Mischung mit einem Schleppmittel auf eine sich über dem Katalysator befindende beheizte Verdampfungszone. Diese Verdampfungszone ist mit einem inerten Material wie Glas- oder Edelstahlkugeln gefüllt. Bei einer Verdampfungstemperatur von 300–500°C gehen die hochsiedenden 3-Alkylamino-2-cyclohexenone in die Dampfphase über. Die Zufuhr eines auf 300–500°C vorgeheizten inerten Trägergases, z.B. Stickstoff, erweist sich zur Erzielung einer vollständigen Verdampfung und einer hohen Selektivität als günstig. Die Zugabe von Wasserstoff zum inerten Trägergas wirkt einem vorzeitigen Verkoken des Katalysators entgegen und ist daher empfehlenswert. Vorzugsweise setzt man 100 bis 500 l Stickstoff und 50 bis 200 l Wasserstoff pro Mol 3-Amino-2-cyclohexenon ein.

Die Verdampfung der Ausgangsverbindung kann auch in der Weise ausgeführt werden, dass seine Mischung mit dem Schleppmittel mit Hilfe eines vorgeheizten Stickstoff- oder Stickstoff/Wasserstoffstromes in einer über dem Katalysator befindlichen Zweistoffdüse versprüht wird.

Die Dehydrierungsreaktion wird bei 200 bis 500°C, vorzugsweise 250 bis 400°C, und einem Druck von 0,1 bis 10 bar, vorzugsweise 0,5 bis 2 bar und insbesondere Normaldruck durchgeführt.

Katalysatoren, die eine längere Zeit eingesetzt werden, lassen im allgemeinen langsam in ihrer Raum-Zeit-Ausbeute nach. Eine der möglichen Ursachen für diese Desaktivierung ist die Abscheidung von Kohlenstoff auf dem Katalysator. Diese Katalysatoren können regeneriert werden durch Abbrennen des Kohlenstoffs in einem Sauerstoff-Stickstoff-Gemisch bei einer Temperatur von 350 bis 500°C. Nach dem Abbrennen des Kohlenstoffs muss der Metallkatalysator wieder durch Überleiten von Wasserstoff reduziert werden.

Folgende Arbeitsweise hat sich zur Durchführung des erfindungsgemässen Verfahrens als besonders günstig erwiesen: Die Umsetzung erfolgt in einem senkrecht stehenden, von aussen beheizbaren Reaktor. Der Katalysator befindet sich in dessen mittlerem Teil. Darüber befindet sich die Verdampfungszone, auf die die Mischung aus Ausgangsverbindung und Schleppmittel getropft wird. Vorgeheizter Stickstoff und Wasserstoff werden von oben über den Katalysator geleitet. Das Reaktionsgemisch kondensiert am unteren Reaktorende. Bei Verwendung von Wasser als Schleppmittel fällt das 3-Alkylaminophenol in kristalliner Form aus und wird abfiltriert. Bei Verwendung organischer Schleppmittel werden die Produkte durch Destillation gewonnen.

Die folgenden Beispiele sollen die Erfindung erläutern; die angegebenen Gasmengen (l/h) sind stets unter Normalbedingungen gemessen.

Beispiel 1

Kohle mit einer spezifischen Oberfläche von ca. 1000 $m^2/g$ wird mit 0,6 Gew.-% Pt durch Imprägnieren mit einer wässrigen Lösung von $K_2PtCl_6$ belegt.

40 ml dieses Katalysators werden in den mittleren Teil eines senkrecht stehenden, von aussen beheizbaren Reaktor gefüllt und bei 330°C durch Überleiten von 20 l/h Stickstoff und 10 l/h Wasserstoff reduziert. Das Ausgangsmaterial 3-Dimethylamino-2-cyclohexenon wird als 50 Gew.-%ige Lösung in Toluol auf die Verdampfungszone oberhalb des Katalysators getropft und in Dampfform mit einer LHSV* von 0,9 $h^{-1}$ bei 330°C zusammen mit 20 l/h Stickstoff und 10 l/h Wasserstoff von oben über den Katalysator geleitet. Die am Unterende des Reaktors erhaltene Produktmischung wird mittels Gaschromatographie analysiert. Bei einem Umsatz von 63% beträgt die Ausbeute an 3-Dimethylaminophenol 7%. Bei Erhöhung der Reaktionstemperatur auf 370°C beträgt die Ausbeute an 3-Dimethylaminophenol 31% bei einem Umsatz von 71%.

*) LHSV (= liquid hourly space velocity) = $\frac{\text{Liter Lösung an Ausgangsverbindung}}{\text{Liter Katalysator} \cdot \text{Stunde}}$

Beispiel 2

Kieselsäure mit einer spezifischen Oberfläche von 100 $m^2/g$ wird mit 1 Gew.-% Pd in Form von Palladiumacetat und 0,9 Gew.-% K in Form von Kaliumhydroxid belegt. 100 ml dieses Katalysators werden in den in Beispiel 1 beschriebenen Reaktor eingefüllt und mit Wasserstoff reduziert. Eine 50 Gew.-prozentige Lösung von 3-Dimethyl-

amino-2-cyclohexenon in Toluol als Schleppmittel wird mit einer LHSV von 0,25 $h^{-1}$ und bei einer Reaktortemperatur von 330°C in Gegenwart von 20 l/h Wasserstoff verdampft und in der Gasphase dehydriert.

Die gaschromatographische Analyse ergibt einen Umsatz von 97% und eine Ausbeute an 3-Dimethylaminophenol von 75%. Weiterhin entstehen 6 Gew.-% Cyclohexanon, 4 Gew.-% Phenol und 3 Gew.-% N,N-Dimethylanilin.

Nach einer Betriebsdauer von etwa 50 Stunden wird der Katalysator durch Abbrennen mit 20 l/h Luft bei 400–450°C regeneriert. Nach der anschliessenden Reduktion des Katalysators wird die gleiche Ausbeute an 3-Dimethylaminophenol erhalten wie vor der Regenerierung. In einem speziellen Versuchslauf wurde eine Katalysatorstandzeit von 1000 Stunden erreicht, wobei 11 mal regeneriert wurde.

Beispiel 3

Es wird der in Beispiel 2 beschriebene Katalysator und Reaktor benutzt. Eine 50 Gew.-%ige wässrige Lösung von 3-Dimethylamino-2-cyclohexenon wird verdampft und in der Gasphase bei 330°C dehydriert. Dabei werden zusätzlich 30 l/h Stickstoff und 10 l/h Wasserstoff durch den Reaktor geleitet. Bei einer LHSV von 0,5 $h^{-1}$ wird vollständiger Umsatz erreicht. Das 3-Dimethylaminophenol fällt aus der wässrigen Produktlösung in Form von weiss-braunen Kristallen aus.

In 52 Stunden Betriebszeit werden 639 g des Einsatzmaterials dehydriert. Nach Trocknung werden 617 g 3-Dimethylaminophenol erhalten, entsprechend einer Ausbeute von 98%. Die Raum-Zeit-Ausbeute beträgt 250 g/l·h. Das Produkt hat einen Schmelzpunkt von 70°C. Anschliessend wird der Katalysator durch Abbrennen mit 10 l/h Luft bei 400–450°C regeneriert und dann wieder reduziert.

Anschliessend wird unter den gleichen Bedingungen wie vor dem Regenerieren aus 804 g Ausgangsmaterial 764 g 3-Dimethylaminophenol, entsprechend 96% Ausbeute, erhalten.

Nach einem nachmaligen Regenerieren des Katalysators werden in 50 Stunden Betriebszeit weitere 595 g 3-Dimethylaminophenol mit einer Ausbeute von 78% erhalten. Der Umsatz beträgt 95%. Das unumgesetzte Einsatzmaterial bleibt im Wasser gelöst, während das Reaktionsprodukt in kristalliner Form anfällt und abfiltriert wird.

Beispiel 4

Es wird der in Beispiel 1 beschriebene Reaktor benutzt. 50 ml eines Katalysators mit 1 Gew.-% Pd und 0,9 Gew.-% K auf Kieselsäure mit einer spezifischen Oberfläche von 100 $m^2/g$ werden zur Dehydrierung von 3-Dimethylamino-2-cyclohexenon eingesetzt. Das Einsatzmaterial wird als 80%ige Lösung in Wasser verdampft. Bei einer Reaktortemperatur von 350°C und einer LHSV von 0,5 $h^{-1}$ werden zusätzlich 20 l/h Stickstoff und 10 l/h Wasserstoff zugeführt. Der Umsatz beträgt 100%. Das 3-Diethylaminophenol fällt in kristalliner Form aus und wird abfiltriert. Die Ausbeute beträgt 80%.

Beispiel 5

Mit dem gleichen Katalysator und unter den gleichen Reaktionsbedingungen wie in Beispiel 4 wird 3-Methylamino-2-cyclohexenon als 40%ige Lösung in Wasser verdampft und in der Gasphase dehydriert. Das Reaktionsprodukt bildet eine wässrige und eine organische Phase. Das umgesetzte 3-Methylamino-2-cyclohexenon verbleibt in der wässrigen Phase; der Umsatz beträgt 95%. Die Ausbeute an 3-Methylaminophenol beträgt nach Destillation im Vakuum 70%.

Beispiel 6

Ebenso wie in Beispiel 5 werden 57 g 3-Dimethylamino-5-methyl-2-cyclohexenon als 50%ige Lösung in Wasser verdampft und in der Gasphase dehydriert. Bei einer LHSV von 0,3 $h^{-1}$ beträgt der Umsatz 100%. Nach Destillation der organischen Phase des Reaktionsproduktes werden 41 g 3-Dimethylamino-5-methylphenol, entsprechend einer Ausbeute von 72%, erhalten.

**Patentansprüche**

1. Verfahren zur Herstellung von 3-Alkylaminophenolen der allgemeinen Formel I:

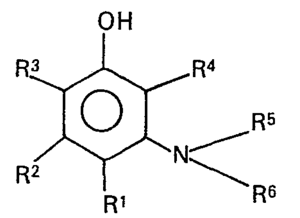
(I)

wobei die Reste $R^1$ bis $R^5$ unabhängig voneinander Wasserstoff, Alkyl- oder Cycloalkylgruppen mit bis zu 12 C-Atomen und gegebenenfalls halogensubstituiert sein können und $R^6$ eine Alkyl- oder Cycloalkylgruppe mit bis zu 12 C-Atomen oder zusammen mit $R^5$ einen Alkylenring mit 4 bis 6 C-Atomen, welcher noch ein Sauerstoff- oder ein substituiertes Stickstoff-Atom enthalten kann, bedeutet, durch katalytische Dehydrierung von 3-Amino-2-cyclohexenonen der allgemeinen Formel II:

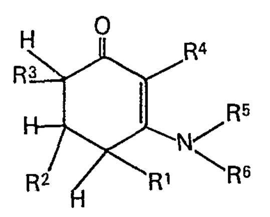
(II)

wobei die Reste $R^1$ bis $R^6$ die oben angegebene Bedeutung haben, durch gekennzeichnet, dass man die Dehydrierung in der Gasphase bei 200 bis 500°C und einem Druck von 0.1 bis 10 bar durchführt.

2. Verfahren nach Anspruch 1, dadurch gekennzeichnet, dass man die Reaktion in Gegenwart von 10 bis 90 Gew.-%, bezogen auf 3-Amino-2-cyclohexenon, eines inerten Lösungsmittels mit einem Siedepunkt zwischen 50 und 300°C durchführt.

3. Verfahren nach Anspruch 2, dadurch gekennzeichnet, dass man Wasser als inertes Lösungsmittel einsetzt.

4. Verfahren nach einem der Ansprüche 1 bis 3, dadurch gekennzeichnet, dass man die Reaktion in Gegenwart von 100 bis 500 l Stickstoff und von 50–200 l Wasserstoff pro Mol 3-Amino-2-cyclohexenon durchführt.

5. Verfahren nach einem der Ansprüche 1 bis 4, dadurch gekennzeichnet, dass man als Katalysator Palladium auf einem Siliciumdioxid- oder Kohle-Träger, mit einem Palladiumanteil von 0.1 bis 20 Gew.-%, einsetzt.

6. Verfahren zur Regenerierung des Dehydrierungskatalysators gemäss Anspruch 5, dadurch gekennzeichnet, dass der Katalysator bei Temperaturen von 350 bis 500°C mit einem Sauerstoff-Stickstoffgemisch und anschliessend bei 250 bis 500°C mit Wasserstoff behandelt wird.

## Claims

1. A process for the preparation of 3-alkylaminophenols of the general formula I

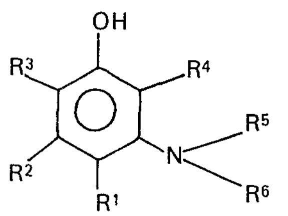

(I)

in which the radicals $R^1$ to $R^5$ independently of one another can be hydrogen or alkyl or cycloalkyl groups having up to 12 C atoms which may optionally be substituted by halogen, and $R^6$ denotes an alkyl or cycloalkyl group having up to 12 C atoms or, together with $R^5$, denotes an alkylene ring which has 4 to 6 atoms and which can also contain an oxygen atom or a substituted nitrogen atom, by catalytic dehydrogenation of 3-amino-2-cyclohexenones of the general formula II

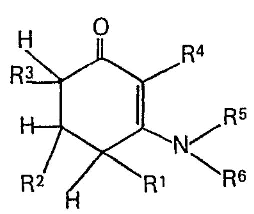

(II)

in which the radicals $R^1$ to $R^6$ have the meaning indicated above, which is characterized by carrying out the dehydrogenation in the gas phase at 200 to 500°C and under a pressure of 0.1 to 10 bar.

2. The process as claimed in claim 1, wherein the reaction is carried out in the presence of 10 to 90% by weight, relative to 3-amino-2-cyclohexenone, of an inert solvent having a boiling point between 50 and 300°C.

3. The process as claimed in claim 2, wherein water is employed as the inert solvent.

4. The process as claimed in any one of claims 1 to 3, wherein the reaction is carried out in the presence of 100 to 500 l of nitrogen and of 50–200 l of hydrogen, per mole of 3-amino-2-cyclohexenone.

5. The process as claimed in any one of claims 1 to 4, wherein the catalyst employed is palladium on a silicon dioxide or carbon support, with a palladium content of 0.1 to 20% by weight.

6. A process for the regeneration of the dehydrogenation catalyst according to claim 5, wherein the catalyst is treated with an oxygen-nitrogen mixture at temperatures of 350 to 500°C and is then treated with hydrogen at 250 to 500°C.

## Revendications

1. Procédé de préparation d'alkylamino-3 phénols répondant à la formule générale I:

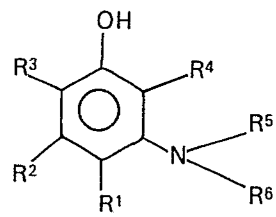

(I)

dans laquelle les symboles $R^1$ à $R^5$ peuvent représenter chacun, indépendamment les uns des autres, un atome d'hydrogène ou un radical alkyl ou cyclo-alkyle contenant au plus 12 atomes de carbone et éventuellement halogéné, et $R^6$ représente un radical alkyle ou cyclo-alkyle contenant au plus 12 atomes de carbone ou forme, avec $R^5$, un radical alkylène appartenant à un cycle contenant de 4 à 6 atomes de carbone et pouvant en outre renfermer un atome d'oxygène ou un atome d'azote substitué, par déshydrogénation catalytique d'amino-3 cyclohexène-2 ones répondant à la formule générale II:

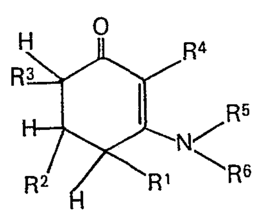

(II)

dans laquelle les symboles $R^1$ à $R^6$ ont les significations qui ont été données ci-dessus, procédé caractérisé en ce qu'on effectue la déshydrogénation en phase gazeuse, à une température de 200 à 500°C et sous une pression de 0,1 à 10 bar.

2. Procédé selon la revendication 1 caractérisé en ce qu'on effectue la réaction en présence de 10 à 90% en poids, par rapport à l'amino-3 cyclohexène-2 one, d'un solvant inerte ayant un point d'ébullition compris entre 50 et 300°C.

3. Procédé selon la revendication 2 caractérisé en ce qu'on utilise l'eau comme solvant inerte.

4. Procédé selon l'une quelconque des revendications 1 à 3, caractérisé en ce qu'on effectue la réaction en présence de 100 à 500 litres d'azote et de 50 à 200 litres d'hydrogène par mole d'amino-3 cyclohexène-2 one.

5. Procédé selon l'une quelconque des revendications 1 à 4, caractérisé en ce qu'on utilise, comme catalyseur, du palladium sur un support de silice ou de charbon, la teneur en palladium étant de 0,1 à 20% en poids.

6. Procédé pour régénérer le catalyseur de déshydrogénation selon la revendication 5, procédé caractérisé en ce qu'on traite le catalyseur à des températures de 350 à 500°C par un mélange d'oxygène et d'azote, puis, à des températures de 250 à 500°C, par de l'hydrogène.